# EUROPEAN PATENT APPLICATION

(11) **EP 4 052 705 A1**
(43) Date of publication of application: **07.09.2022**
(21) Application number: 21161105.8
(22) Date of filing: 05.03.2021
(51) Int. Cl.: A61K 31/4245, A61K 31/498, A61K 31/47, A61K 31/00, A61P 31/22, C12N 15/113

(54) **COMPOSITIONS FOR THE TREATMENT OF EBV ASSOCIATED DISEASES OR CONDITIONS**

(71) Applicant: Universität Basel Vizerektorat Forschung, 4001 Basel (CH)
(72) Inventor: HESS, Christoph, 4051 Basel (CH); MÜLLER-DUROVIC, Bojana, 5000 Aarau (CH); BANTUG, Glenn R., 4127 Birsfelden (CH)
(74) Representative: Cooley (UK) LLP

(57) **Abstract**

The invention relates to the treatment and prevention of diseases and conditions associated with EBV infection. In particular, the invention is directed to the use of an IDO1 inhibitor for the treatment and prevention of diseases and conditions associated with EBV infection.

## Description

### Field

The disclosure is directed to the treatment and prevention of diseases and conditions associated with EBV infection. In particular, the disclosure is directed to the use of an IDO1 inhibitor for the treatment and prevention of diseases and conditions associated with EBV infection.

### Background

Epstein-Barr virus (EBV) is a γ-herpesvirus that primarily infects B cells and human epithelial cells. The prominent hallmark of herpesviruses is the capacity to readily establish lifelong infection (latency) in their host, with EBV establishing latency mainly in B lymphocytes. In a latent state, herpesviruses usually do not produce disease. Based on seroprevalence, 95% of adults carry EBV world-wide. The virus has a well-established oncogenic potential and is associated with ∼ 1% of all human cancers and can cause a broad range of diseases ranging from lymphoproliferative diseases, inflammatory immune dysregulations, epithelial cancers to autoimmune diseases (Farrell, P. J. (2019) Annu. Rev. Pathol. Mech. Dis. 14, 29-53; Wald A. & Corey L. (2007) Herpesviruses; Biology, Therapy and Immunoprohylacis, Cambridge University Press; Zhang, T. et al. (2014) Pathology - Research and Practice 210, 69-73).

Primary infection mostly occurs in childhood and is asymptomatic but can also manifest as infectious mononucleosis (IM) when primary infection occurs in the adolescent. IM is the most common clinical manifestation upon EBV infection.

The lifecycle of EBV encompasses three different phases, pre-latent phase, latent phase and lytic phase. Upon infection of naïve B cells, the virus does not induce its *de novo* synthesis but initiates the pre-latent phase, during which a subset of viral lytic genes together with latent genes is expressed. The EBV DNA acquires a repressive epigenetic signature pattern during this phase leading to the eventual silencing of all lytic genes but also certain latent genes. This process of epigenetic shutoff of transcription is completed about ten to 14 days post-infection and is followed by the latent phase of infection.

The virus remains latent in an episomal state, which is characterized by the expression of a small subset of genes. The different sets of viral genes expressed in latently infected cells are termed EBNAs (Epstein-Barr nuclear antigens) and LMPs (latent membrane proteins) together with noncoding transcripts such as viral microRNAs and long noncoding RNAs.

Periodically, the virus may become reactivated from the latent state through mechanisms that are unclear. In this lytic phase of infection, all lytic genes of EBV (>80 genes) are expressed, potent viral DNA replication takes place and progeny virus particles are produced. In immunocompetent hosts, CD4+ and CD8+ T cells, especially cytotoxic CD8+ T cells, are effective at controlling this process. In contrast, reactivation is clinically significant in immunocompromised patients (e.g. after stem cell or organ transplantation, in patients treated for autoimmunity or cancer, in the setting of HIV/AIDS or immunodeficiencies) leading to the development of lymphomas such as Burkitt's lymphoma (BL) and Hodgkin's lymphoma (HL) and being associated with EBV associated immune dysregulation, for example manifesting as haemophagocytosis syndrome.

Immunosuppressive therapy during hematopoietic stem cell transplantation (HSCT) or solid organ transplantation (SOT) is strongly associated with EBV associated malignancies. One of the deadliest risks post-transplantation is the development of post-transplant lymphoproliferative disorder (PTLD). Most cases of PTLD are B cell lymphomas and up to 5% are T cell lymphomas, Hodgkin, or Hodgkin-like lymphomas. EBV plays a major role in the pathogenesis of PTLD, particularly in early lesions. Early PTLD is usually reported within the first-year post transplantation, with the majority of cases occurring within the first 6 months. Incidence in HSCT ranges from 1% to 11% depending on the type of transplant and degree of immune suppression and peaks 2-3 months post-engraftment. During SOT, the incidence ranges from 0.5% to 20% also depending on the type of transplant and the immunosuppressive regime with a median onset of 6 months. Recipients of renal grafts, bone marrow grafts, and stem cell grafts have a low frequency of PTLD (1% or less) and those with heart-lung/lung grafts or intestinal grafts the highest. Pediatric patients have the most significant risk of developing PTLD since they are often EBV-naïve prior to transplantation and at risk of acquiring the virus from EBV-positive grafts.

In addition, immunodeficiencies are linked with severe and an often fatal course of EBV infection, including but not limited to: Ataxia-Telangiectasia, ITK deficiency, X-linked lymphoproliferative disease (XLP), Wiskott-Aldrich syndrome, CD27 deficiency, XMEN disease (MAGT1 deficiency), Coronin 1a deficiency, autoimmune lymphoproliferative syndrome (ALPS), *MST1* mutation (STK4 deficiency), Omenn syndrome, DiGeorge syndrome, Activated PI3K-δ syndrome, WHIM syndrome, CTPS1 deficiency, MCM4 deficiency, ZAP70 deficiency and NF-κB1 haploinsufficiency. Immunodeficiencies facilitate virus reactivation and uncontrolled proliferation of EBV-infected B lymphocytes and the eventual development of an EBV associated lymphoproliferative disease.

Further complications upon EBV infection include chronic active EBV (CAEBV), which is a rare syndrome characterized by prolonged IM-like symptoms and elevated peripheral blood EBV DNA load in apparently immunocompetent persons. The prognosis of CAEB is generally poor and HSCT is the only curative therapy. In addition, EBV infection can result in a haemophagocytic syndrome (HPS), hemophagocytic lymphohistiocytosis and immune haemolytic anemias.

EBV infection has also been linked with various autoimmune disorders that might arise as immunopathologic consequences of long-term virus carriage (e.g., multiple sclerosis, systemic lupus erythematosus, rheumatoid arthritis, inflammatory bowel disease).

EBV associated tumors can further arise in clinically immunocompetent hosts (e.g., Hodgkin's lymphoma (HL), diffuse large B cell lymphoma, Burkitt's lymphoma (BL), gastric carcinoma, nasopharyngeal carcinoma, T/NK cell lymphoma).

Therapy for IM focuses on relieving symptoms. Nonsteroidal anti-inflammatory drugs (NSAIDs) are given to reduce inflammation, headaches and muscle pain (e.g. ibuprofen, naproxen and acetaminophen).

PTLD treatment can be challenging. The aim is to cure PTLD while preserving the function of the transplanted organ. The first line treatment is a reduction of immunosuppressive medication to the lowest possible dose. In case the reduction of immunosuppression is not sufficient, additional treatment might be needed. Rituximab, a chimeric monoclonal antibody against CD20, is a possible treatment option, which depletes hyperproliferative CD20+ B cells. In case the before-mentioned therapies fail, CHOP chemotherapy is an additional therapy of choice (doxorubicin, cyclophosphamide, vincristine, prednisone). Rituximab and CHOP chemotherapy can also be combined, known as R-CHOP. Occasionally, surgery or radiotherapy may also be used to treat PTLD. Adoptive T cell therapy involves the treatment with EBV-specific T cells and is used in patients who have not responded to other treatment options. Several targeted drugs are studied in clinical trials for their effectiveness to treat PTLDs and include cell signal blockers such as ibrutinib, idelalisib, proteasome inhibitors such as bortezomib, radioimmunotherapy such as 90Y-ibritumomab tiuxetan, checkpoint inhibitors such as pembrolizumab and nivolumab and antibody-drug conjugates such as brentuximab vedotin. These treatment modalities can also be used for immunodeficiencies associated with uncontrolled EBV infection and CAEBV.

No EBV-specific vaccine or EBV-specific anti-viral drug has been approved for patient treatment to date.

There remains a need in the art for therapeutics that target EBV, EBV infection and diseases or conditions associated with EBV. Specifically, there remains a need in the art for therapeutics that target mechanisms of EBV infection and the spread of EBV infection and diseases or conditions associated with such processes. It is a further object of the invention to provide an improved treatment strategy for the diseases described herein that targets EBV and its lifecycle.

### Summary of the Invention

In a first aspect, the invention provides an Indoleamine 2,3-dioxygenase 1 (IDO1) inhibitor for use in a method of treating an Epstein-Barr virus (EBV) associated disease or condition in a subject.

In another aspect, the invention provides a method of treating an EBV associated disease or condition as defined herein in a subject in need thereof, comprising administering to the subject a therapeutically effective amount or a prophylactically effective amount of an IDO1 inhibitor as defined herein or a composition comprising an IDO1 inhibitor as defined herein.

### Brief description of the Figures

Figure 1 shows a schematic overview of the Kynurenine pathway (KP) and interlinked NAD⁺ *de novo* biosynthesis.
Figures 2A-2B show how EBV infection of B cells causes accumulation of Quinolinate (QUIN) and depletion of L-Tryptophan (L-TRYP) and NAD⁺.

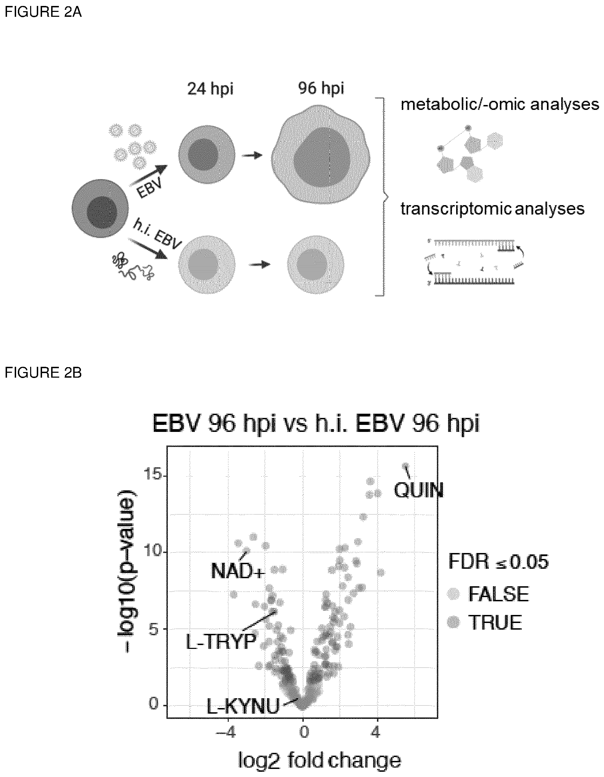

Figure 2A shows an *experimental scheme* - B cells were either infected with EBV or exposed to an identical amount of EBV previously heat inactivated (h.i. EBV).
Figure 2B shows a volcano plot depicting metabolite abundance in EBV-infected vs. h.i. EBV exposed primary human B cells (n=6).

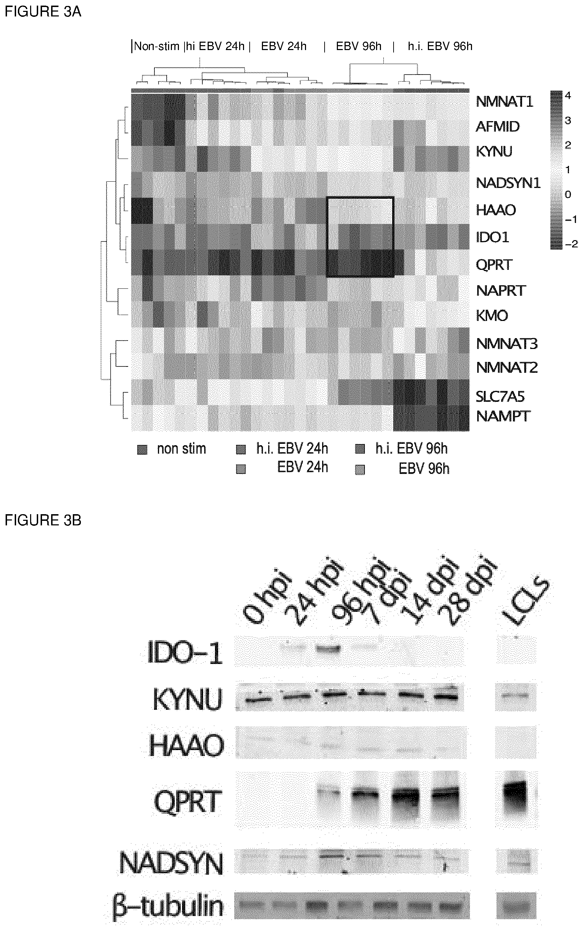

Figures 3A-3E show how EBV infection of B cells induces the Kynurenine pathway.
Figure 3A shows a heatmap depicting relative expression of transcripts encoding for Tryptophan metabolism genes in uninfected, EBV-infected and h.i. EBV exposed B cells, at 24 and 96 hpi (n=6).
Figure 3B shows representative Western blot of total Indoleamine 2,3-dioxygenase 1 (IDO1), Kynureninase (KYNU), 3-Hydroxyanthranilate 3,4-dioxygenase (HAAO), quinolinate phosphoribosyltransferase (QPRT) and NAD⁺ synthetase (NADSYN) abundance during early EBV infection (up to 96 hpi), as well as during outgrowth of lymphoblastoid cell lines (LCL).

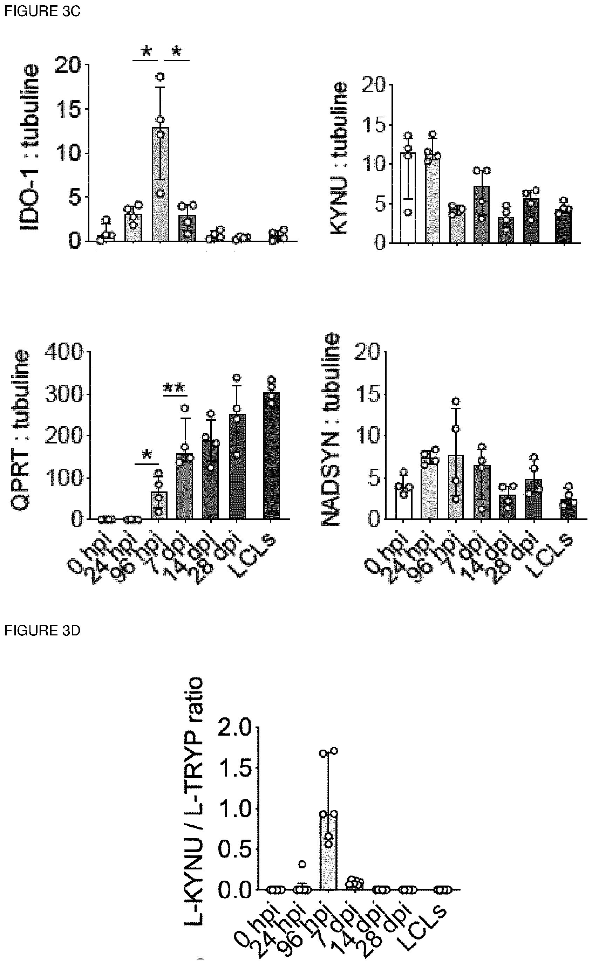

Figure 3C shows expression of the enzymes IDO1, KYNU, QPRT and NADSYN, normalized to β-tubulin, in B cells during early EBV infection and LCL outgrowth (n=4).
Figure 3D shows the ratio of L-Kynurenine (L-KYNU) to L-Tryptophan (L-TRYP) during early EBV infection and LCL outgrowth, analyzed by LCMS/MS (n=6).

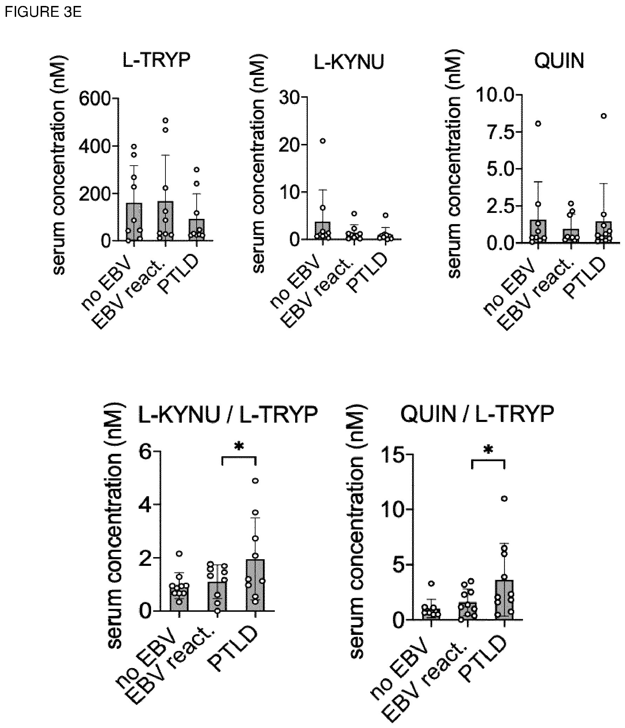

Figure 3E shows L-TRYP, L-KYNU and QUIN serum concentrations in solid organ transplant recipients were measured by LCMS/MS (n=10, each). L-KYNU/L-TRYP and QUIN/L-TRYP ratios were calculated based on single metabolite abundance.

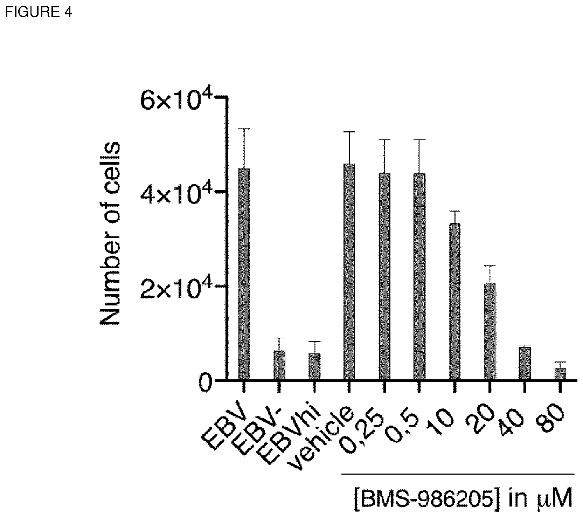

Figure 4 shows how EBV-induced IDO-1 activity is required for B cell proliferation. Proliferation was analyzed in the presence of vehicle or different BMS-986205 concentrations added on day 0 post EBV-infection of primary B cells (n=2). Uninfected and h.i. EBV infected cells served as internal controls. Proliferation was analyzed 8 days post infection by flow cytometry using a cell trace violet based assay.

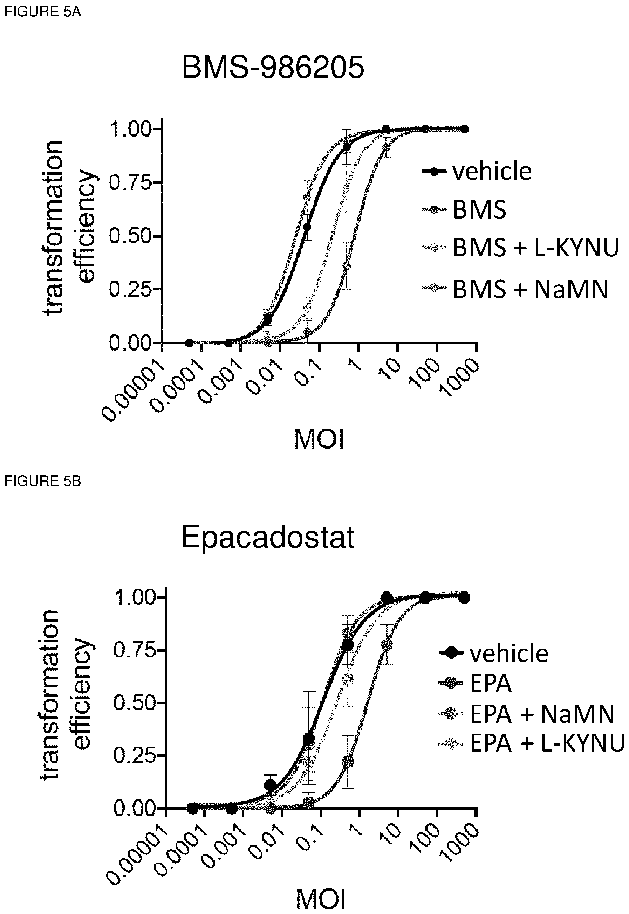

Figures 5A-5B show how EBV-induced IDO-1 activity is required for B cell transformation.
Figure 5A shows a transformation efficiency assay analyzed in the presence of vehicle, 10µM BMS-986205, 10µM BMS-986205 / 50µM L-KYNU, or 10µM BMS-986205 / 500µM NaMN added on day 0 post EBV-infection of primary B cells (n=3). Transformation efficiency was analyzed 5 weeks post infection and plotted against the added multiplicity of infection (MOI).
Figure 5B shows a transformation efficiency assay analyzed in the presence of vehicle, 100µM Epacadpostat, 100µM Epacadpostat / 500µM NaMN, or 100µM Epacadpostat / 100µM L-KYNU added on day 0 post EBV-infection of primary B cells (n=3). Transformation efficiency was analyzed 5 weeks post infection and plotted against the added multiplicity of infection (MOI).

### Detailed description

The invention described herein is based upon the identification of a metabolic vulnerability of EBV in its capacity to establish latent infection in newly infected B cells.

Transient indoleamine 2,3-dioxygenase 1 (IDO1) expression was identified as a signature metabolic adaptation associated with early EBV infection of B cells. This IDO1 expression was found to be virus-initiated, specifically via EBNA-2. Importantly, early transient IDO1 activity in newly EBV-infected B cells was identified as a metabolic requirement of EBV's capacity to establish latent infection of B cells. Accordingly, EBV-driven B cell transformation can be efficiently suppressed by inhibiting IDO1 activity in nascently EBV-infected B cells. B cell proliferation can also be suppressed by inhibiting IDO1 activity in nascently EBV-infected B cells.

Inhibition of IDO1 activity, for example with an IDO1 inhibitor, can therefore be used to prevent newly EBV-infected B cells from becoming latently infected and transformed (i.e., immortalized) by EBV.

III-controlled EBV infection, with interlinked expansion of the pool of latently EBV-infected B cells via infection by EBV virions derived from a lytic infection component, is associated with numerous diseases: On the one hand, primary infection with EBV (for example, infectious mononucleosis) can be associated with high abundance of infectious units (EBV virions) in plasma/serum, a lytic infection component and severe and prolonged clinical signs and symptoms. In patients with primary immunodeficiencies (for example, XLP) primary EBV infection can be fatal. On the other hand, once latent infection and a balance between the virus and the immune system is established, immunodeficiencies (both primary and secondary) or immunosuppression facilitate virus reactivation, including a lytic infection component. Spread of EBV virions via a lytic infection component to previously uninfected B cells drives expansion of the pool of latently infected B cells, which again can drive both immune pathology and facilitate development of EBV associated lymphoproliferative diseases (from benign polyclonal lymphoproliferative diseases to malignant lymphoproliferative diseases, for example).

The invention thus concerns the identification of a novel target for pharmacological intervention for the treatment of an EBV associated disease or condition. Specifically, the methods of the invention concern the prevention of latent EBV infection of B cells, and thus the treatment of diseases associated with ill-controlled or uncontrolled EBV infection. In particular, the invention provides a therapeutic approach that targets IDO1 to treat or prevent diseases that are linked to ill-controlled or uncontrolled EBV infection with a lytic component (i.e., diseases that are, at least partially, underpinned by the spread of EBV virions to non-infected B cells, where EBV establishes latent infection).

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred embodiments of compositions, methods and materials are described herein.

### IDO1 inhibitors

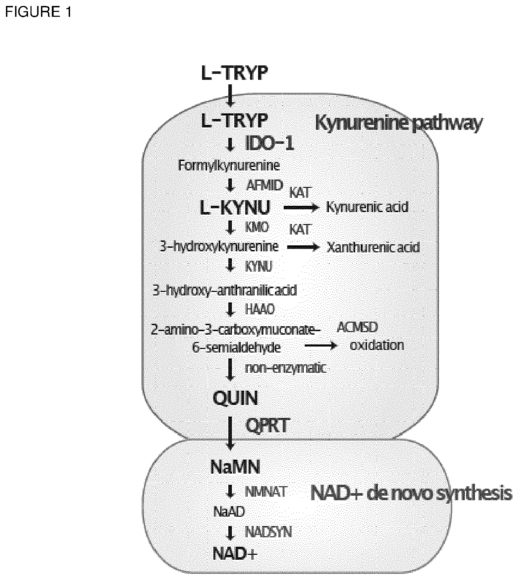

Indoleamine 2,3-dioxygenase 1 (IDO1) is an intracellular enzyme that catalyses the first and rate-limiting step of the kynurenine pathway (KP), the major route of tryptophan degradation in the human (see Figure 1). It depletes local tryptophan (L-TRYP) concentration leading to increasing concentrations of downstream metabolites, including L-Kynurenine (L-KYNU). Beside IDO1, Indoleamine 2,3-Dioxygenase 2 (IDO2) or Tryptophan-2,3-Dioxygenase (TDO) also catalyze this reaction. While TDO is mainly expressed in the liver, IDO1 is expressed in various human tissues including several types of immune cells.

IDO1 is overexpressed by cancer cells and antigen presenting dendritic cells in the tumor microenvironment (TME). Enhanced IDO1 activity in the TME depletes local tryptophan and produces L-Kynurenine, which induces T cell anergy and suppresses tumor control by the immune system. IDO1 has been described in the literature as playing an important role in evasion of immunosurveillance by cancer cells. As such, the IDO1 signalling pathway has been a target for the development of cancer immunotherapies.

IDO1 inhibitors are currently being investigated in clinical trials for treating cancers. The most promising data from such studies relate to the combination of an IDO1 inhibitor with immune checkpoint inhibitors such as pembrolizumab and nivolumab, which inhibit the programmed death 1 (PD-1) pathway in T cells.

In contrast, the invention concerns the identification of EBV-induced IDO1 expression in B cells as a virus-driven metabolic adaption in the course of early infection. Specifically, EBV-induced transient IDO1 activity in newly infected B cells is a metabolic requirement to establish latent EBV infection. Pharmacological inhibition of IDO1 activity can efficiently supress EBV-driven B cell transformation, for example with an IDO1 inhibitor as described herein.

IDO1 inhibitors are known in the art (see Cheong, J, E. et al. (2018) Expert Opinion on Therapeutic Patents, 2018, 28:4, 317-330, which is incorporated herein by reference).

Examples of IDO1 inhibitors according to the invention include IDO1 inhibitors disclosed in the following documents, all of which are incorporated herein by reference:

### Small molecule inhibitors

WO2010005958, WO2015070007, WO2017079669, WO2017152857, WO2017129139, WO2017106062, WO2017002078, US20160333009, WO2017024996, WO2016027241, WO2018140831, WO2017181849, WO2016073770, WO2016073738, WO2016073774, WO2016071283, WO2016026772, WO2014081689, WO2015173764, WO2016181348, WO2016181349, WO2015082499, WO2015150097, WO2016071293, WO2017133258, WO2017007700, WO2016161960, WO2017034420, WO2016024233, WO2012142237, WO2014159248, WO2016051181, WO2016169421, WO2016165613, WO2016037026, WO2016059412, WO2017140274, WO2017075341, WO2017149469, WO2017134555, WO2013069765, US2013065905, US20150352106, WO2017010106, WO2015002918, WO2015006520, WO2015031295, WO2015006520, WO2014150646, WO2014150677, WO2016210414, WO2016161269, WO2016161279, WO2016161286, WO2017051353, WO2017051354, WO2017139414, WO2014186035, WO2016201354, WO2018140831

### vaccines

WO2017149150 shRNA

Phan, T. et al. (2020) Cancer Gene Ther 27:3-4, 235-245. https://pubmed.ncbi.nlm.nih.gov/30824815/

### A) Small molecule IDO1 inhibitors

Currently, there are several small molecule IDO1 inhibitors in clinical development. An IDO1 inhibitor according to the invention can be selected from any one of the following or a pharmaceutically acceptable salt thereof:

### (1) hydroxyamidines, such as the clinical candidate epacadostat

An IDO1 inhibitor according to the invention can be an IDO1 inhibitor containing a hydoxyamidine moiety.

Epacadostat is a representative example and is described in WO2010005958, WO2015070007 and WO2017079669. Clinical trials involving epacadostat include: NCT03361865, NCT03374488, NCT03182894, NCT03322540, NCT03291054, NCT03361228, NCT02364076, NCT03217669, NCT03322566, NCT03832673, NCT04231864, NCT03516708, NCT03325465, NCT03432676, NCT03196232, NCT02298153, NCT03358472, NCT03463161, NCT03328026, NCT03491579, NCT01685255, NCT01961115, NCT03342352, NCT03310567, NCT03402880, NCT03006302, NCT02752074, NCT03444649, NCT03238638, NCT03592407, NCT03348904, NCT03823131, NCT03085914, NCT02042430, NCT03414229, NCT03602586, NCT03347123, NCT02318277, NCT03532295, NCT01604889, NCT01982487, NCT02862457, NCT02327078, NCT03322384, NCT02575807, NCT01822691, NCT02959437, NCT03493945, NCT02118285, NCT02166905, NCT02178722, NCT01195311, NCT03277352, NCT02785250, NCT02559492, NCT03589651, NCT03471286, NCT04463771, NCT04586244, and NCT03707457.

An IDO1 inhibitor according to the invention can be

A compound of Formula I: 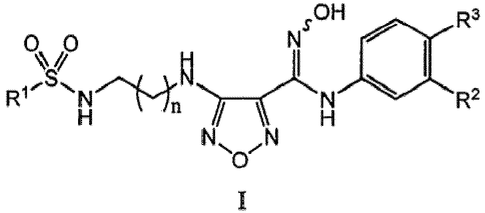 or a pharmaceutically acceptable salt thereof, wherein: R¹ is NH₂ or CH₃; R² is CI, Br, CF₃, CH₃, or CN; R³ is H or F; and n is 1 or 2.

An IDO1 inhibitor according to the invention can be an epacadostat derivative as disclosed in WO2017152857, WO2017129139, WO2017106062, and WO2017002078.

An IDO1 inhibitor according to the invention can be an IDO1 inhibitor as disclosed in US20160333009 (Gilead).

An IDO1 inhibitor according to the invention can be an IDO1 inhibitor as disclosed in WO2017024996 (Hengrui Medicine), preferably HTI-1090, for example as disclosed in NCT03208959.

An IDO1 inhibitor according to the invention can be an IDO1 inhibitor as disclosed in WO2016027241, WO2018140831, suitably RG-70099 (Curadev/Roche).

An IDO1 inhibitor according to the invention can be selected from any one of: 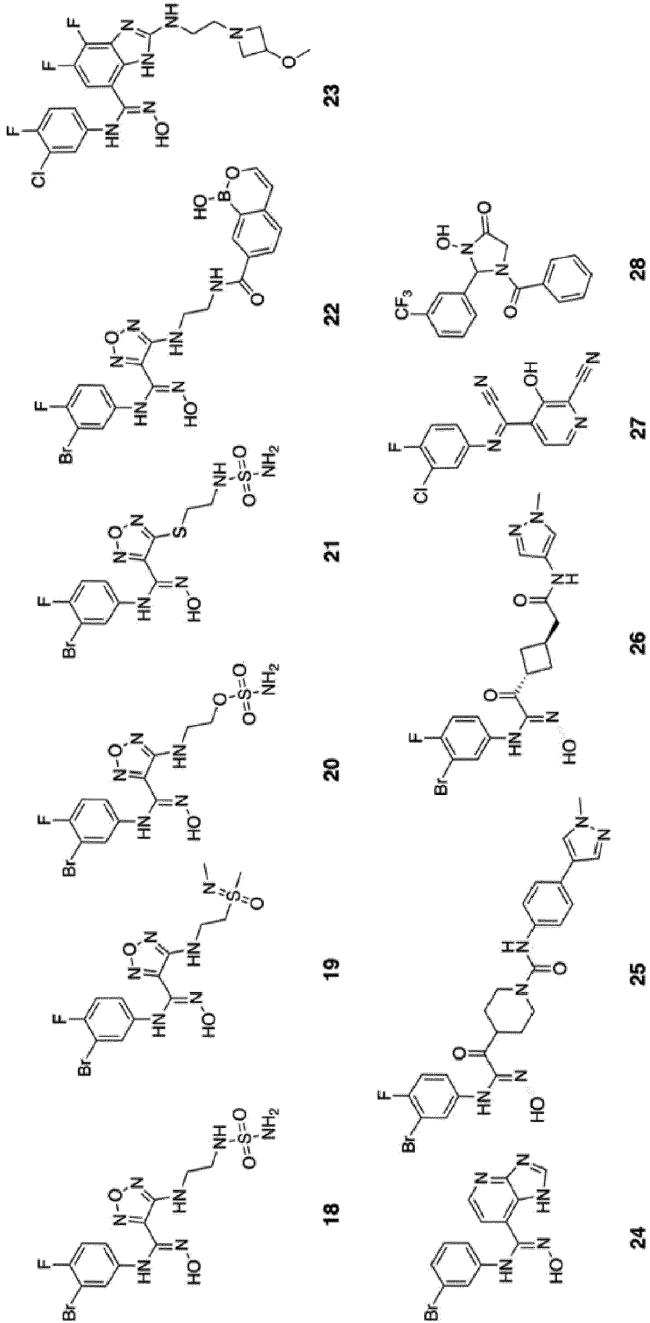 or a pharmaceutically acceptable salt thereof.

An IDO1 inhibitor according to the invention can be selected from epacadostat, HTI-1090, RG-70099 and pharmaceutically acceptable salts thereof. In a preferred embodiment, an IDO1 inhibitor according to the invention is epacadostat or a derivative thereof or a pharmaceutically acceptable salt thereof.

### (2) BMS-986205 and others

An IDO1 inhibitor according to the invention can be a 1-(4-arylcyclohex-1-yl)propenamide.

BMS-986205 (Linrodostat) is a representative example and is described in WO2017181849, WO2016073770, WO2016073738 and WO2016073774. Clinical trials involving BMS-986205 include: NCT03936374, NCT03378310, NCT03312426, NCT03374228, NCT04106414, NCT03695250, NCT03329846, NCT03362411, NCT03792750, NCT03247283, NCT03661320, NCT03346837, NCT03192943, NCT02658890, NCT03386838, NCT03417037, NCT03519256, NCT04007588, NCT03854032, NCT04047706, NCT03459222, NCT02996110, NCT02750514, NCT02935634, and NCT03335540.

An IDO1 inhibitor according to the invention can be a compound of the formula: 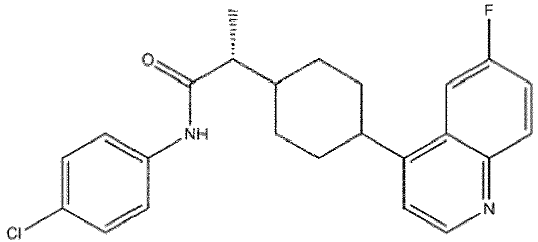 or a pharmaceutically acceptable salt thereof.

An IDO1 inhibitor according to the invention can be an IDO1 inhibitor as disclosed in WO2016071283 and WO2016026772 (IOMet).

An IDO1 inhibitor according to the invention can be an IDO1 inhibitor as disclosed in WO2014081689 (Vertex).

An IDO1 inhibitor according to the invention can be selected from any one of: 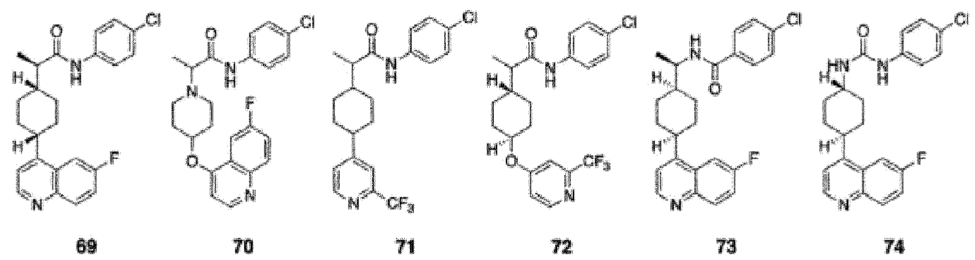 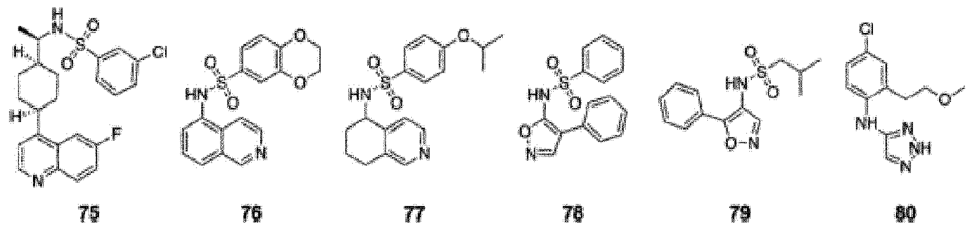 or a pharmaceutically acceptable salt thereof.

In a preferred embodiment, an IDO1 inhibitor according to the invention is BMS-986205 or a derivative thereof or a pharmaceutically acceptable salt thereof.

### (3) Indoles and [5,6] heterocyclic arenes, such as the clinical candidates indoximod and PF-06840003

An IDO1 inhibitor according to the invention can be an Indole and [5,6]-fused heteroaromatic. Indoximod (1-methyl-D-tryptophan) is a representative example and was developed by NewLink Genetics. Indoximod has advanced into clinical development for the treatment of cancer. However, it has also been acknowledged that indoximod is not an IDO1 inhibitor and does not inhibit IDO1 enzyme activity. Clinical trials involving indoximod include: NCT01560923, NCT02835729, NCT02502708, NCT02077881, NCT03301636, NCT00739609, NCT02073123, NCT02460367, NCT01042535, NCT01792050, NCT03372239, NCT03852446, NCT00567931, NCT04049669, NCT02052648, NCT01191216, NCT01302821, NCT04755608, NCT03165318, NCT04379674, and NCT02913430.

An IDO1 inhibitor according to the invention can be an indol-3-yl-pyrrolidine-2,5-dione as disclosed in WO2015173764 or the clinical candidate PF-06840003 (EOS-200271) as disclosed in WO2016181348 and WO2016181349. Clinical trials involving PF-06840003 include: NCT02764151.

An IDO1 inhibitor according to the invention can be a 4-(indol-3-yl)-3,6-dihydro-2H-pyridine as disclosed in WO2015082499 (IOMet).

An IDO1 inhibitor according to the invention can be an indole-2-carboxamide as disclosed in WO2015150097.

An IDO1 inhibitor according to the invention can include indazoles as disclosed in WO2016071293, WO2017133258, imidazo[1,5-a]pyridine as disclosed in WO2017007700 and WO2016161960.

An IDO1 inhibitor according to the invention can be a [1,2]-Oxaxolo[5,4-b]pyridine as disclosed in WO2016024233 and WO2017034420.

An IDO1 inhibitor according to the invention can be selected from any one of: 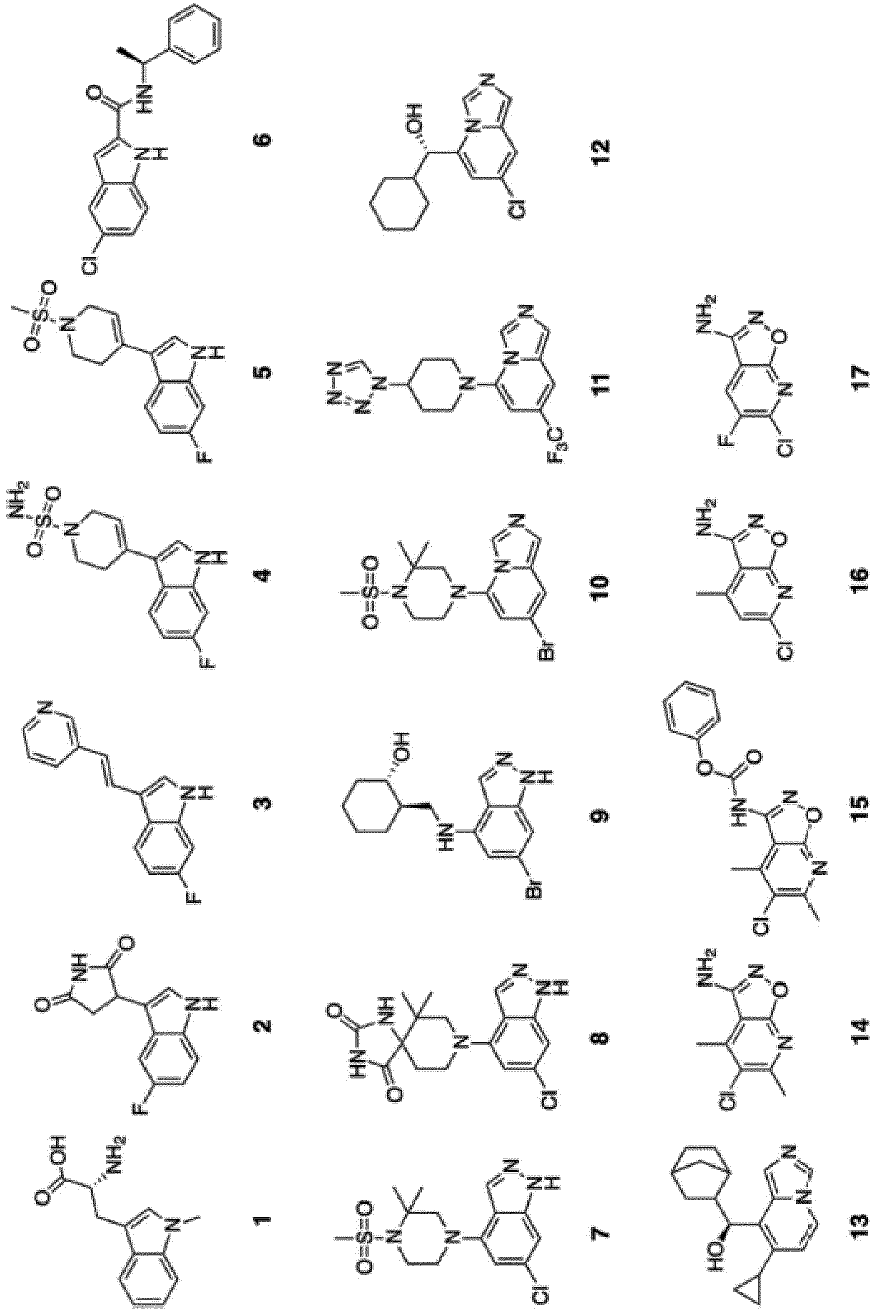 or a pharmaceutically acceptable salt thereof.

### (4) 4-phenylimidazoles (4-Pls), such as the clinical candidate navoximod

An IDO1 inhibitor according to the invention can be a 4-phenylimidazole (4-PI). The clinical candidate navoximod is a representative example, as disclosed in WO2012142237 (Newlink). Clinical trials involving navoximod include: NCT02471846 and NCT02048709.

An IDO1 inhibitor according to the invention can be an isomeric imidazoleindoles as disclosed in WO2014159248 and WO2016051181.

An IDO1 inhibitor according to the invention can be a N-[(4-pyrazol-4-yl)phenyl]piperidine substituted imidazoleisoindole derivative as disclosed in WO2016169421 (Hengrui Medicine).

An IDO1 inhibitor according to the invention can be an imidazoleisoindoles substituted with a bridged bi-/tri-cyclic group as disclosed in WO2016165613 (Innogate Pharma).

An IDO1 inhibitor according to the invention can be a derivative of navoximod, as disclosed in WO2016037026 (Merck).

An IDO1 inhibitor according to the invention can be an IDO1 inhibitor as disclosed in WO2016059412 (Redx Pharma).

An IDO1 inhibitor according to the invention can be an IDO1 inhibitor as disclosed in WO2017140274.

An IDO1 inhibitor according to the invention can be a an IDO1 inhibitor as disclosed in WO2017075341 (Scifluor Life Sciences), WO2017149469 and WO2017134555.

An IDO1 inhibitor according to the invention can be selected from any one of: 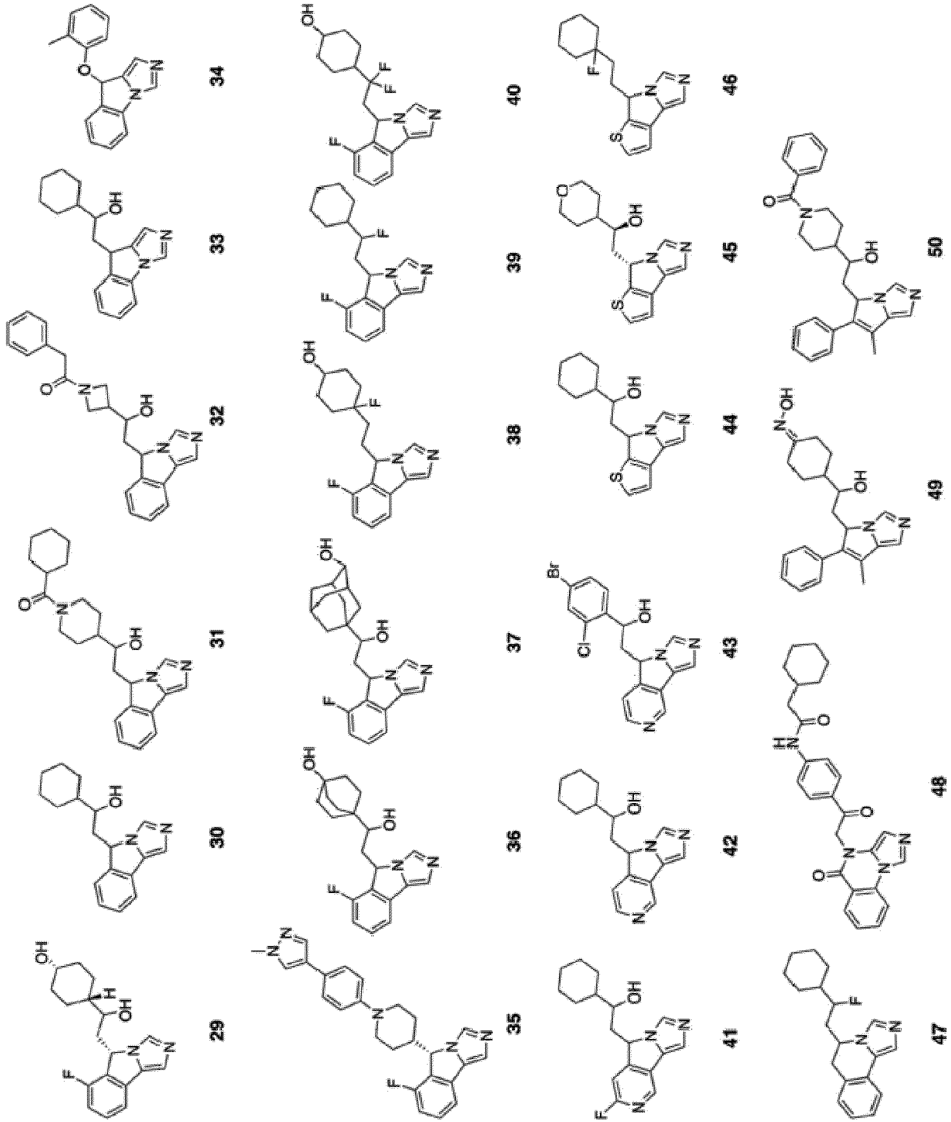 or a pharmaceutically acceptable salt thereof.

### (5) 1, 2-diamino- substituted and 1-hydroxy-2-amino-substituted arenes, including the clinical candidate KHK2455

An IDO1 inhibitor according to the invention can be a derivative of 2-alkyoxy-3-aminoquinoxaline, such as the clinical candidate KHK2455 (Kyowa Hakko Kirin), as disclosed in the following clinical trials: NCT04321694, NCT03915405, and NCT02867007.

An IDO1 inhibitor according to the invention can be a quinoxaline substituted with ortho arylmethoxy and sulfonamido, or any of the IDO1 inhibitors disclosed in WO2013069765, US2013065905, US20150352106 and WO2017010106.

An IDO1 inhibitor according to the invention can be 1-alkoxy-2-ureido-biphenyl as disclosed in WO2015002918; aryl-1,2-diamines as disclosed in WO2015006520, WO2015031295 and WO2015006520; ureido monoaryl-1,2-diamines as disclosed in WO2014150646, WO2014150677 and WO2016210414; and monoaryl-1,2-diamines as disclosed in WO2016161269, WO2016161279, and WO2016161286 (BMS).

An IDO1 inhibitor according to the invention can be a an IDO1 inhibitor as disclosed in WO2017051353 and WO2017051354 (GSK).

An IDO1 inhibitor according to the invention can be an aryl-1,2-diamine as disclosed in WO2017139414 (InventisBio).

An IDO1 inhibitor according to the invention can be a an ortho-diamino substituted furo[2,3-c]pyridine or thieno[2,3-c]pyridines as disclosed in WO2014186035 (Curadev).

An IDO1 inhibitor according to the invention can be selected from any one of: 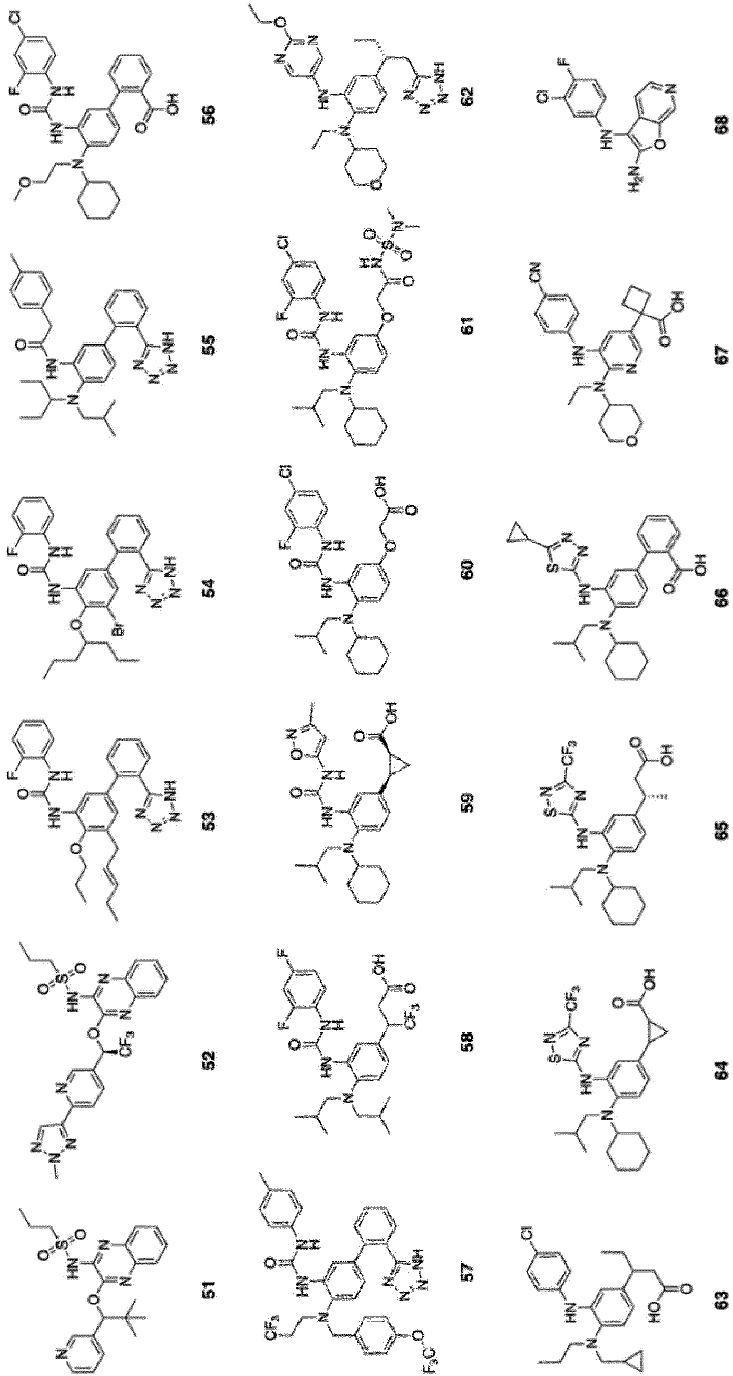 or a pharmaceutically acceptable salt thereof.

### (6) Others

An IDO1 inhibitor according to the invention can be selected from LY-01013 (Luye Pharma Group Ltd), as disclosed in clinical trial: NCT03844438; MK-7162 (Merck & Co Inc), as disclosed in clinical trial: NCT03364049; GBV-1028 as disclosed in WO2016201354; TPST-8844 (Tempest Therapeutics Inc); BGB-5777 (BeiGene); IOM2983 (Merck/IOMet); RG-70099 (Curadev/Roche); and HTI-1090 (SHR9146) (Jiangsu HengRui Medicine Co., Ltd.).

The term "small molecule" encompasses numerous biological and chemical classes, including synthetic, semi-synthetic, or naturally-occurring inorganic or organic molecules, including synthetic, recombinant or naturally-occurring compounds. A "small molecule" also refers to an agent that has a molecular weight of less than about 5 kD, less than about 4 kD, less than about 3 kD, less than about 2 kD, less than about 1 kD, or less than about 0.5kD. Small molecules can be obtained from a combinatorial small organic molecule library containing a large number of potential therapeutic compounds. Such "combinatorial chemical libraries" or "ligand libraries" can be screened separately or screened in pools, to identify those library members of a particular chemical species or subclasses that display the desired characteristic activity of inhibiting IDO1 activity.

The present invention includes salts of the IDO1 inhibitors described herein. As used herein, "salts" refers to derivatives of the disclosed compounds wherein the parent compound is modified by converting an existing acid or base moiety to its salt form. Examples of salts include, but are not limited to, mineral acid (such as HCI, HBr, H₂SO₄) or organic acid (such as acetic acid, benzoic acid, trifluoroacetic acid) salts of basic residues such as amines; alkali (such as Li, Na, K, Mg, Ca) or organic (such as trialkylammonium) salts of acidic residues such as carboxylic acids; and the like. The salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile (ACN) are preferred.

The term "pharmaceutically acceptable salt" used herein includes a subset of the "salts" described above which are, conventional non-toxic salts of the parent compound formed, for example, from non-toxic inorganic or organic acids. Lists of suitable salts are found in Remington's Pharmaceutical Sciences, 17th ed., Mack Publishing Company, Easton, Pa., 1985, p. 1418 and Journal of Pharmaceutical Science, 66, 2 (1977), each of which is incorporated herein by reference in its entirety. "Pharmaceutically acceptable" is a term used herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

A small molecule IDO1 inhibitor according to the invention can be an IDO1 inhibitor according to the definition understood by those skilled in the art. In a preferred aspect, an IDO1 inhibitor can be a molecule, such as a small molecule IDO1 inhibitor as disclosed herein, that inhibits IDO1 enzyme activity according to assays known in the art. In a preferred aspect, an IDO1 inhibitor can be a molecule, such as a small molecule IDO1 inhibitor as disclosed herein, that binds to IDO1 and inhibits IDO1 enzyme activity according to assays known in the art. An IDO1 inhibitor can be a molecule, such as a small molecule IDO1 inhibitor as disclosed herein, preferably a small molecule IDO1 inhibitor as disclosed herein that inhibits IDO1 enzyme activity, that has any one or more of the following IDO1 binding characteristics:
(i) reversible and competitive inhibitor,
(ii) irreversible inhibitor.

Preferably, an IDO1 inhibitor according to the invention is a reversible and competitive inhibitor of IDO1, such as epacadostat.

Preferably, an IDO1 inhibitor according to the invention is an irreversible inhibitor of IDO1, such as BMS-986205.

An IDO1 inhibitor according to the invention can inhibit IDO1 enzyme activity with an IC50 of about 1µM or less, preferably about 100nM or less, preferably about 10nM or less, preferably about 1nM or less.

An IDO1 inhibitor according to the invention can inhibit IDO1 activity in cell-based assay with an IC50 of about 100µM or less, preferably about 10µM or less, preferably about 1µM or less, preferably about 100nM or less, preferably about 10nM or less, preferably about 1nM or less.

An IDO1 inhibitor according to the invention can exhibit at least 10-fold selectivity for binding IDO1 over TDO, preferably at least 20-, 30-, 40-, 50-, 60-, 70-, 80-, 90- or 100-fold selectivity for binding IDO1 over TDO, preferably at least 100-fold.

An IDO1 inhibitor can be a molecule, such as a small molecule IDO1 inhibitor as disclosed herein, preferably a small molecule IDO1 inhibitor as disclosed herein that inhibits IDO1 enzyme activity and:
a) inhibits L-TRYP to L-KYNU conversion in B cells, preferably nascent EBV-infected B cells, according to the assays described herein;
b) inhibits B cell proliferation, preferably EBV-induced B cell proliferation, according to the assays described herein;
c) inhibits B cell transformation, preferably EBV-induced B cell transformation, according to the assays described herein; or
d) any one or more of a)-c) above, preferably a), b) and c) above.

An IDO1 inhibitor according to the invention can inhibit L-TRYP to L-KYNU conversion in B cells. Preferably, an IDO1 inhibitor according to the invention can inhibit L-TRYP to L-KYNU conversion in nascent EBV-infected B cells. L-TRYP and L-KYNU levels can be analysed by methods known in the art, for example mass spectrometry (e.g. LCMS/MS). Alternatively, L-TRYP and L-KYNU levels could be detected using an ELISA or any other suitable assay. An IDO1 inhibitor according to the invention can be any one of the IDO inhibitors as disclosed herein that can inhibit L-TRYP to L-KYNU conversion in B cells, preferably nascent EBV-infected B cells.

An IDO1 inhibitor according to the invention can inhibit B cell proliferation. Preferably, an IDO1 inhibitor according to the invention can inhibit EBV-induced B cell proliferation. Proliferation of B cells can be analysed by methods known in the art, for example using a commercially available Cell trace proliferation kit (*e.g.,* a CFSE proliferation kit).

Alternatively, proliferation can be determined using a commercially available cell proliferation kit (*e.g.,* a BrdU incorporation assay) or any other suitable assay. Suitably, an IDO1 inhibitor according to the invention can inhibit B cell proliferation with an IC50 of about 100µM or less, about 50µM or less, about 20µM or less, about 15µM or less, about 10µM or less, about 5µM or less, about 1µM or less, or about 100nM or less in the assays described herein, preferably about 10µM or less. An IDO1 inhibitor according to the invention can be any one of the IDO inhibitors as disclosed herein that can inhibit B cell proliferation, preferably EBV-induced B cell proliferation.

An IDO1 inhibitor according to the invention can inhibit B cell transformation. Preferably, an IDO1 inhibitor according to the invention can inhibit EBV-induced B cell transformation. Transformation can be analysed by methods known in the art, for example using a transformation efficiency assay. In this assay B cells are seeded into a cell culture plate and infected with increasing virus concentrations. An IDO1 inhibitor can be added immediately after infection. After an incubation period of 5 weeks, the number of wells positive for LCL outgrowth are counted. Alternatively, any other suitable assay can be used. Suitably, an IDO1 inhibitor according to the invention can inhibit B cell transformation at a concentration of about 200µM or less, about 150µM or less, about 100µM or less, about 50µM or less, about 20µM or less, about 15µM or less, about 10µM or less, about 5µM or less, or about 1µM or less, preferably about 100µM or less or about 10µM or less in the assays described herein. An IDO1 inhibitor according to the invention can be any one of the IDO inhibitors as disclosed herein that can inhibit B cell transformation, preferably EBV-induced B cell transformation.

In one aspect, an IDO1 inhibitor according to the invention, preferably a small molecule IDO1 inhibitor as disclosed herein that inhibits IDO1 enzyme activity, can inhibit L-TRYP to L-KYNU conversion in B cells, preferably nascent EBV-infected B cells as described herein, and inhibit B cell proliferation, preferably EBV-induced B cell proliferation as described herein.

In one aspect, an IDO1 inhibitor according to the invention, preferably a small molecule IDO1 inhibitor as disclosed herein that inhibits IDO1 enzyme activity, can inhibit L-TRYP to LKYNU conversion in B cells, preferably nascent EBV-infected B cells as described herein, and inhibit B cell transformation, preferably EBV-induced B cell transformation as described herein.

In one aspect, an IDO1 inhibitor according to the invention, preferably a small molecule IDO1 inhibitor as disclosed herein that inhibits IDO1 enzyme activity, can inhibit B cell proliferation, preferably EBV-induced B cell proliferation as described herein and inhibit B cell transformation, preferably EBV-induced B cell transformation as described herein.

In one aspect, an IDO1 inhibitor according to the invention, preferably a small molecule IDO1 inhibitor as disclosed herein that inhibits IDO1 enzyme activity, can inhibit L-TRYP to L-KYNU conversion in B cells, preferably nascent EBV-infected B cells as described herein; inhibit B cell proliferation, preferably EBV-induced B cell proliferation as described herein; and inhibit B cell transformation, preferably EBV-induced B cell transformation as described herein.

### B) vaccines

An IDO1 inhibitor according to the invention can be a vaccine. A representative example is 10102 (IO-Biotech), as disclosed in WO2017149150. An immunotherapeutic composition comprising an adjuvant and an immunogenic fragment of IDO1, for example an immunogenic fragment which consists of up to 25 consecutive amino acids of the sequence of IDO1.

### C) shRNA

An IDO1 inhibitor according to the invention can be a shRNA. A representative example is shIDO-ST (Tara Immuno-Oncology; City of Hope) as disclosed in Phan, T. et al. (2020) Cancer Gene Ther 27:3-4, 235-245 (https://pubmed.ncbi.nlm.nih.gov/30824815/) or a shRNA as disclosed in US2017081671.

### Compositions

An IDO1 inhibitor according to the invention can be provided as a composition, for example a pharmaceutical composition comprising an IDO1 inhibitor as described herein and at least one pharmaceutically acceptable excipient. Therapeutic or pharmaceutical compositions may comprise other components such as a carrier, vehicle, excipients, carriers or vehicles.

Compositions described herein include, but are not limited to, pharmaceutical compositions. A "pharmaceutical composition" refers to a formulation of a composition with one or more pharmaceutically acceptable carriers, diluents or excipients generally accepted in the art for the delivery of a compound or drug to a mammal, e.g., humans. In particular embodiments, pharmaceutical compositions can comprise an IDO1 inhibitor formulated with one or more pharmaceutically acceptable carriers, diluents, and/or excipients. It will also be understood that, if desired, the compositions of the invention may be administered in combination with other agents as well, such as, e.g., nucleic acids, proteins, small molecules, or pharmaceutically-active agents, adjunct therapies, etc. so long as the desired therapeutic effect is achieved.

In particular embodiments, compositions can comprise pharmaceutically acceptable formulations with therapeutically effective amounts of anIDO1 inhibitor as described herein or derivatives thereof; or prodrugs, solvates, stereoisomers, racemates, or tautomers of IDO1 inhibitors formulated with one or more pharmaceutically acceptable carriers (additives), other active agents, and/or diluents.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. As used herein "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, surfactant, or emulsifier which has been approved by drug approval authorities, for example the United States Food and Drug Administration, as being acceptable for use in humans or domestic animals. Exemplary pharmaceutically acceptable carriers include, but are not limited to, to sugars, such as lactose, glucose and sucrose; starches, such as corn starch and potato starch; cellulose, and its derivatives, such as sodium carboxymethyl cellulose, ethyl cellulose and cellulose acetate; tragacanth; malt; gelatin; talc; cocoa butter, waxes, animal and vegetable fats, paraffins, silicones, bentonites, silicic acid, zinc oxide; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and any other compatible substances employed in pharmaceutical formulations.

Methods of formulating compositions are known to the skilled artisan and are described in the following: Physicians Desk Reference, 62nd edition. Oradell, NJ: Medical Economics Co., 2008; Goodman & Gilman's The Pharmacological Basis of Therapeutics, Eleventh Edition. McGraw-Hill, 2005; Remington: The Science and Practice of Pharmacy, 20th Edition. Baltimore, MD: Lippincott Williams & Wilkins, 2000; and The Merck Index, Fourteenth Edition. Whitehouse Station, NJ: Merck Research Laboratories, 2006; each of which is hereby incorporated by reference in relevant parts.

### Combinations

An IDO1 inhibitor as described herein can be administered in combination with one or more additional therapeutic agent or modality.

Compositions described herein can comprise an effective amount of an IDO1 inhibitor alone or in combination with one or more other therapeutic agents or modalities. The compositions may be administered alone or in combination with other known treatments for the diseases disclosed herein. Exemplary therapeutic agents or modalities include:
Immunosuppressants, such as calcineurin inhibitors (*e.g*. tacrolimus and cyclosporine; mTOR inhibitors *(e.g.* sirolimus); purine antagonists, IL2R antagonists, corticosteroids *(e.g.* methylprednisolone, dexamethasone, prednisone), antiproliferative agents (e.g. Mycophenolate Mofetil, Mycophenolate Sodium, Azathioprine, cyclophosphamide);
Anti-inflammatory agents and analgesics, such as nonsteroidal anti-inflammatory drugs (NSAIDs), ibuprofen, naproxen and acetaminophen;
Therapeutic agents for PTLD, such as Rituximab; CHOP chemotherapy (doxorubicin, cyclophosphamide, vincristine, prednisone); Rituximab and CHOP chemotherapy (R-CHOP); cell signal blockers such as ibrutinib, idelalisib; proteasome inhibitors such as bortezomib; radioimmunotherapy such as 90Y-ibritumomab tiuxetan; checkpoint inhibitors such as pembrolizumab and nivolumab; and antibody-drug conjugates such as brentuximab vedotin.

Antiviral agents, such as ganciclovir; valganciclovir, aciclovir; Cancer treatments, such as radiation therapy, chemotherapy, transplantation, immunosuppressant therapy, immunotherapy, hormone therapy, photodynamic therapy; Immunodeficiency therapies and autoimmune therapies.

### Treatment

The invention provides an IDO1 inhibitor as described herein or compositions comprising the same for use in a method of treating a disease or condition as described herein.

The invention also provides a method of treating a disease or condition as described herein comprising administering to a subject in need thereof a therapeutically effective amount or a prophylactically effective amount of an IDO1 inhibitor as described herein or composition comprising an IDO1 inhibitor as described herein.

Any of the IDO1 inhibitors or compositions described herein can be used in any of the methods described herein.

The terms "treating," "treatment", and the like are used herein to generally mean obtaining a desired pharmacologic and/or physiologic effect. The effect may be prophylactic in terms of completely or partially preventing a disease or condition and/or may be therapeutic in terms of a partial or complete cure for a disease or condition and/or adverse effect attributable to the disease. "Treatment" as used herein covers any treatment of a disease or condition in a mammal, and includes: ameliorating a disease, disorder or condition (*i.e*., slowing or arresting or reducing the development of the disease, disorder or condition or at least one of the clinical symptoms thereof); alleviating or ameliorating at least one physical parameter including those which may not be discernible by the patient; modulating the disease, disorder or condition, either physically, (*e.g*., stabilization of a discernible symptom), physiologically, (*e.g*., stabilization of a physical parameter), or both; or preventing or delaying the onset or development or progression of the disease, disorder or condition or one or more clinical symptoms thereof.

As used herein, the phrase "ameliorating at least one symptom of" refers to decreasing one or more symptoms of the disease or condition for which the subject is being treated. The disease or condition being treated can be selected from any of the diseases or conditions disclosed herein, preferably post-transplant lymphoproliferative disorder (PTLD), Infectious Mononucleosis (IM) or glandular fever, chronic active EBV (CAEBV), haemophagocytic syndrome (HPS), hemophagocytic lymphohistiocytosis, immune haemolytic anemias, an EBV associated cancer, an EBV associated disease or condition in an immunodeficient subject, or an EBV associated autoimmune disease. In one aspect, the disease is PTLD and the one or more symptoms ameliorated include, but are not limited to, lymphadenopathies, fever, fatigue, weight loss, night sweats and general malaise. In one aspect, the disease is IM and the one or more symptoms ameliorated include, but are not limited to, lymphadenopathies in neck and armpits, fatigue, fever, soft and swollen spleen, headache, swollen tonsils and skin rash.

As used herein, "prevent," and similar words such as "prevented," "preventing" etc., indicate an approach for preventing, inhibiting, or reducing the likelihood of the occurrence or recurrence of a disease or condition. It also refers to delaying the onset or recurrence of a disease or condition or delaying the occurrence or recurrence of the symptoms of a disease or condition. As used herein, "prevention" and similar words also include reducing the intensity, effect, symptoms and/or burden of a disease or condition prior to onset or recurrence of the disease or condition.

A "therapeutically effective amount" of an IDO1 inhibitor may vary according to factors such as the disease state, age, sex, and weight of the individual, and the agent to elicit a desired response in the individual. A therapeutically effective amount is also one in which any toxic or detrimental effects of the agent are outweighed by the therapeutically beneficial effects. The term "therapeutically effective amount" includes an amount that is effective to "treat" a subject (e.g., a patient).

A "prophylactically effective amount" refers to an amount of an IDO1 inhibitor effective to achieve the desired prophylactic result. As a prophylactic dose may be used in subjects prior to or at an earlier stage of disease, the prophylactically effective amount can be less than the therapeutically effective amount.

A method of treating a subject as described herein can comprise administering to a subject in need thereof a therapeutically effective amount or a prophylactically effective amount of an IDO1 inhibitor as described herein or composition comprising an IDO1 inhibitor as described herein. Compositions described herein may be administered as one or more solids, semi-solids, gels, or liquids, or combination thereof. For example, an IDO1 inhibitor may be individually formulated for intravenous administration in a liquid dosage form or for oral administration as a single tablet or capsule or as a combination of one or more tablets, capsules, or other dosage forms. The specific amount/dosage regimen will vary depending on the weight, gender, age, and health of the individual; the formulation, the biochemical nature, bioactivity, bioavailability and the side effects of the IDO1 inhibitor and the number and identity of agents in the complete therapeutic regimen.

As used herein, the terms "administering," "administer," or "administration" refer to the delivery of one or more compounds or compositions to a subject parenterally, enterally or topically. Illustrative examples of parenteral administration include, but are not limited to, intravenous, intramuscular, intraarterial, intrathecal, intracapsular, intraorbital, intracardiac, intradermal, intra peritoneal, transtracheal, subcutaneous, subcuticular, intraarticulare, subcapsular, subarachnoid, intraspinal, and intrasternal injection and infusion. Illustrative examples of enteral administration include, but are not limited to oral, inhalation, intranasal, sublingual, and rectal administration. Illustrative examples of topical administration include, but are not limited to, transdermal and vaginal administration.

Administration can include administration of a composition or formulation that includes the IDO1 inhibitor or composition as described herein and one or more additional therapeutic agent, or the essentially simultaneous, sequential or separate administration of separate formulations of the IDO1 inhibitor or composition and one or more additional therapeutic agent.

### EBV associated diseases and conditions

Numerous diseases and conditions are associated with EBV infection.

In one aspect, a method of treating a disease or condition as described herein can comprise treating an EBV associated disease or condition in a subject. In one aspect, an IDO1 inhibitor or composition as described herein is for use in a method of treating an EBV associated disease or condition in a subject. Preferably, the disease or condition is associated with an EBV infection.

An EBV associated disease or condition as described herein can comprise a disease associated with any one or more of the following:
a) ill-controlled or uncontrolled EBV infection in a subject;
b) latent EBV infection with a lytic EBV component in a subject;
c) uncontrolled proliferation of B cell lymphocytes latently infected with EBV in a subject.

Suitably, an EBV associated disease is an EBV associated lymphoproliferative disease.

Measuring peripheral whole blood or plasma EBV DNA load in a subject in any of the methods described herein can identify a disease or condition as an EBV associated disease or condition. In a preferred aspect, an EBV associated disease or condition as described herein comprises a disease or condition associated with an EBV DNA load in a subject of greater than or equal to about 5,000 copies/µg DNA in blood and/or an EBV DNA load of greater than or equal to about 1,000 copies/100 µl plasma. In a preferred aspect, an EBV associated disease or condition as described herein comprises a disease associated with an EBV DNA load in a subject that is increasing over time. EBV DNA load can be measured using techniques known in the art.

In one aspect, an EBV associated disease or condition as described herein comprises an EBV infection. An EBV infection can be a primary EBV infection, a latent EBV infection or a latent EBV infection with a lytic EBV component.

A method of treating a disease or condition as described herein can comprise treating a primary EBV infection. Suitably, a method of treating a disease or condition as described herein can comprise treating Infectious Mononucleosis (IM) or glandular fever, chronic active EBV (CAEBV), haemophagocytic syndrome (HPS), hemophagocytic lymphohistiocytosis and immune haemolytic anemias.

An IDO1 inhibitor as described herein or a composition comprising the same can be used in a method of treating a primary EBV infection selected from IM, CAEBV, HPS, hemophagocytic lymphohistiocytosis and immune haemolytic anemias.

In one aspect, a method of treating a primary EBV infection comprises administering the IDO1 inhibitor or composition as described herein when the first clinical signs of an EBV infection occur. An IDO1 inhibitor as described herein can prevent EBV naive B cells from becoming latently infected, thus limiting the expansion of the pool of latently infected cells.

Transplant patients are at risk of developing post-transplant lymphoproliferative disorder (PTLD) during the course of immunosuppressive medication.

In one aspect, an EBV associated disease or condition as described herein comprises PTLD. A method of treating a disease or condition as described herein can comprise a method of treating PTLD in a subject. Preferably, the method of treating an EBV associated disease comprises treating PTLD in a transplant patient.

EBV naive transplant patients, typically pediatric patients, are at risk of primary EBV infection from EBV-positive allogeneic grafts. In one aspect, a method of treating a disease or condition as described herein can comprise a method of preventing a primary EBV infection in a subject. In one aspect, an IDO1 inhibitor or composition as described herein is for use in a method of preventing a primary EBV infection in a subject, preferably an EBV naive patient, preferably an EBV naïve transplant patient. In one aspect, a method of treating an EBV associated disease or condition as described herein comprises preventing a primary EBV infection or PTLD in an EBV naïve transplant patient.

The risk of a subject developing PTLD can depend on the type of transplant and the immunosuppressive regime.

The transplant can be a hematopoietic stem cell transplant (HSCT) or a solid organ transplant (SOT). The transplant can be selected from one or more of a renal, bone marrow, stem cell, heart, lung and intestinal transplant; preferably a heart, lung or intestinal transplant. In various aspects, the transplant patient is receiving an allogeneic transplant.

In various aspects, the transplant patient can be receiving one or more immunosuppressive agent, for example one or more immunosuppressive agent selected from calcineurin inhibitors *(e.g.* tacrolimus and cyclosporine); mTOR inhibitors *(e.g.* sirolimus); purine antagonists; IL2R antagonists; corticosteroids (*e.g*. methylprednisolone, dexamethasone, prednisone); antiproliferative agents (*e.g*. Mycophenolate Mofetil, Mycophenolate Sodium, Azathioprine, cyclophosphamide). High dosages of immunosuppressive agents are associated with higher risk of PTLD. Dosage ranges of immunosuppressive agents are known in art and can be monitored in individual patients.

An IDO1 inhibitor or composition as described herein can be administered, optionally in combination with one or more additional therapeutic agent or modality, to a subject in need of a transplant. In one aspect, an IDO1 inhibitor or composition as described herein can be administered to a subject in need of a transplant concurrently with an immunosuppressive regime associated with the transplant procedure, for example any of the immunosuppressive agents known in the art or described herein.

An IDO1 inhibitor or composition as described herein, optionally in combination with one or more additional therapeutic agent, can be administered to a subject in need of a transplant prior to, concurrently with and/or after receiving a transplant.

Numerous cancers are linked with EBV infection (Farrell, P. J. (2019) Annu. Rev. Pathol. Mech. Dis. 14, 29-53; Wald A. & Corey L. (2007) Herpesviruses).

In one aspect, an EBV associated disease or condition as described herein comprises an EBV associated cancer in a subject. In one aspect, an IDO1 inhibitor or composition as described herein is for use in a method of treating an EBV associated cancer in a subject. An EBV associated cancer can be characterised by uncontrolled proliferation of B cell lymphocytes latently infected with EBV. An EBV associated cancer can be an EBV-positive cancer, for example a cancer characterised by EBV-positive cells, for example greater than or equal to about 50%, 60%, 70%, 75%, 80%, 85%, 90% or 95% of the cancer cells are EBV positive, preferably greater than about 90% of the cancer cells are EBV positive. Cancer cells can be obtained and tested for EBV by methods known in the art, for example detected by EBER *in situ* hybridization (see Zhang, T. et al. (2014) Pathology - Research and Practice 210, 69-73).

An EBV associated cancer can be selected from a lymphoma, preferably derived from B cells; or a carcinoma. In a preferred aspect, an EBV associated cancer is a lymphoma, preferably derived from B cells.

An EBV associated cancer can be a lymphoma selected from immunoblastic lymphomas, for example in people who are immunosuppressed; Burkitt's lymphoma, for example in areas where malaria is hyperendemic; Hodgkin's lymphoma; NK cell lymphoma; T cell lymphoma; diffuse large B cell lymphoma; and primary effusion lymphoma.

An EBV associated cancer can be a carcinoma selected from nasopharyngeal carcinoma and gastric carcinoma, preferably gastric carcinoma.

In one aspect, an IDO1 inhibitor or composition as described herein is for use in a method of treating an EBV associated cancer in a subject in need thereof. Suitably the EBV associated cancer is selected from immunoblastic lymphoma, Burkitt's lymphoma, Hodgkin's lymphoma, NK cell lymphoma, T cell lymphoma, diffuse large B cell lymphoma, primary effusion lymphoma.

Numerous immunodeficiencies are linked with severe and often fatal course of EBV infection. Immunodeficiencies facilitate EBV reactivation, uncontrolled proliferation of EBV-infected B lymphocytes and the eventual development of an EBV associated lymphoproliferative disease.

An EBV associated disease or condition as described herein can comprise a disease or condition in an immunodeficient subject. In one aspect, an IDO1 inhibitor or composition as described herein is for use in a method of treating an EBV associated disease or condition in an immunodeficient subject.

An EBV associated disease or condition in an immunodeficient subject can be selected from Ataxia-Telangiectasia, ITK deficiency, X-linked lymphoproliferative disease (XLP), Wiskott-Aldrich syndrome, CD27 deficiency, XMEN disease (MAGT1 deficiency), Coronin 1a deficiency, autoimmune lymphoproliferative syndrome (ALPS), *MST1* mutation (STK4 deficiency), Omenn syndrome, DiGeorge syndrome, Activated PI3K-δ syndrome, WHIM syndrome, CTPS1 deficiency, MCM4 deficiency, ZAP70 deficiency and NF-κB1 haploinsufficiency.

In one aspect, an IDO1 inhibitor or composition as described herein is for use in a method of treating an treating an EBV associated disease or condition in an immunodeficient subject selected from Ataxia-Telangiectasia, ITK deficiency, X-linked lymphoproliferative disease (XLP), Wiskott-Aldrich syndrome, CD27 deficiency, XMEN disease (MAGT1 deficiency), Coronin 1a deficiency, autoimmune lymphoproliferative syndrome (ALPS), *MST1* mutation (STK4 deficiency), Omenn syndrome, DiGeorge syndrome, Activated PI3K-δ syndrome, WHIM syndrome, CTPS1 deficiency, MCM4 deficiency, ZAP70 deficiency and NF-κB1 haploinsufficiency.

Numerous autoimmune disorders have been linked to the immunopathologic consequence of long-term EBV virus carriage.

An EBV associated disease or condition as described herein comprises an EBV associated autoimmune disease or condition in a subject. In one aspect, an IDO1 inhibitor or composition as described herein is for use in a method of treating an EBV associated autoimmune disease or condition in a subject.

An EBV associated autoimmune disease or condition can be selected from multiple sclerosis, systemic lupus erythematosus, rheumatoid arthritis and inflammatory bowel disease.

In one aspect, an IDO1 inhibitor or composition as described herein is for use in a method of treating an EBV associated autoimmune disease or condition selected from multiple sclerosis, systemic lupus erythematosus, rheumatoid arthritis and inflammatory bowel disease.

### Subject

A subject is in need of a treatment if the subject would benefit biologically, medically or in quality of life from such treatment. Treatment will typically be carried out by a physician who will administer a therapeutically effective amount or a prophylactically effective amount of the IDO1 inhibitor or composition as described herein. Preferably the subject is a human subject. For example, a subject may be suffering from a disease as disclosed herein that has been diagnosed by a clinician based on clinical parameters for the disease. A subject may be suffering from a condition as disclosed herein, for example a condition associated with one or more symptoms of the diseases or conditions disclosed herein but not necessarily meeting one or more clinical parameters for a disease diagnosis.

In a preferred aspect, in any of the methods described herein, a subject has an EBV infection. In a preferred aspect, a subject is latently infected with EBV. EBV infection in a subject can be determined using methods known in the art.

In a preferred aspect, in any of the methods described herein, a subject has a long-term EBV infection. A subject can have an EBV infection for about 6 months or longer, about 9 months or longer, about 1 year or longer, about 2 years or longer, about 3 years or longer.

In any of the methods described herein, a subject can have an EBV DNA load of greater than or equal to about 5,000 copies/µg DNA in blood and/or greater than or equal to about 1,000 copies/100 µl plasma. In any of the methods described herein, the EBV DNA load in a subject in need of a treatment as described herein can be increasing over time. EBV DNA load can be measured using techniques known in the art.

In any of the methods described herein, the subject in need of treatment can be an immunocompromised subject, and preferably a subject having an EBV infection as described herein. In one aspect, an IDO1 inhibitor or composition as described herein is for use in a method of treating or preventing an EBV associated disease in an immunocompromised subject, preferably a subject having an EBV infection as described herein.

In various aspects, an immunocompromised subject can be a subject having a primary or secondary immunodeficiency. Secondary immunodeficiency can result from malnutrition, aging, particular medications (e.g., chemotherapy, disease-modifying antirheumatic drugs, immunosuppressive drugs, glucocorticoids) and environmental toxins like mercury and other heavy metals, pesticides and petrochemicals like styrene, dichlorobenzene, xylene, and ethylphenol. Secondary immunodeficiency can be caused by disease such as cancer, particularly those of the bone marrow and blood cells (e.g., leukemia, lymphoma, multiple myeloma), and infections, such as chronic infections, particularly viral infections such as HIV, SARS-COV, and measles. Secondary immunodeficiency can result from various hormonal and metabolic disorders such as anemia, hypothyroidism and hyperglycemia.

In any of the methods described herein, a subject in need of a treatment as described herein can be a subject exhibiting symptoms of any of the diseases disclosed herein, preferably a subject having an EBV infection as described herein and/or a subject having a diagnosis of any of the diseases disclosed herein, preferably a subject having an EBV infection as described herein.

In any of the methods described herein, a subject in need of a treatment as described herein can be a subject having a diagnosis of PTLD. Diagnosis of PTLD can be according to methods known in the art, for example based on one or more of histological examination of biopsy tissues with most lesions showing malignant B cells, CT images showing enlarged lymph nodes or focal mass, PET scan identifying increase metabolic active (PET avid) lesions.

In one aspect, an IDO1 inhibitor or composition as described herein is for use in a method of treating or preventing PTLD in an immunocompromised subject, preferably a subject receiving one or more immunosuppressive drugs. In one aspect, the subject can be EBV naive, and the treatment preferably comprises prevention of PTLD.

In any of the methods described herein, a subject in need of a treatment as described herein can be a subject exhibiting one or more symptoms of PTLD, for example one or more symptoms selected from lymphadenopathies, fever, fatigue, weight loss, night sweats and general malaise.

In any of the methods described herein, a subject in need of a treatment as described herein can be a subject having a diagnosis of IM. Diagnosis of IM can be according to methods known in the art.

In any of the methods described herein, a subject in need of a treatment as described herein can be a subject exhibiting symptoms of IM, for example one or more symptoms selected from lymphadenopathies in neck and armpits, fatigue, fever, soft and swollen spleen, headache, swollen tonsils and skin rash.

All of the features described herein (including any accompanying claims, abstract and drawings), and/or all of the steps of any method so disclosed, may be combined with any of the above aspects in any combination, except combinations where at least some of such features and/or steps are mutually exclusive. Specifically, any of the active agents and compositions described herein can be used in any of the described methods of treatment. Any and all such combinations are explicitly envisaged as forming part of the invention.

### Examples

### Example 1 - EBV infection and B cell metabolism

Transcriptomic and metabolomic profiling was performed to investigate how infection of B cells with EBV affects their metabolism

Specifically, naïve B cells (CD27⁻ IgD⁺) were purified from buffy coat preparations of healthy blood donors (HDs) and infected with EBV wild-type strain B95.8 via spinoculation, at a concentration optimized to yield ≥98% of infected cells in each experiment. Heat-inactivated EBV (h.i. EBV) served as a control for non-infection related activation of B cells through pathogen associated molecular patterns (PAMPs), and was added at the same concentration as the wild type strain. B cells were then analyzed at 0,24 and 96 hours post-infection (hpi) with EBV, or exposure to h.i. EBV, respectively **(****Figure 2A****;** *Experimental scheme*). The 24 h and 96 h time points represent distinct phases of pre-latent EBV infection: at 24 hpi, extensive transcriptional changes are noted that precede phenotypical and functional changes. At 96 hpi, B cells acquire a lymphoblastoid phenotype, they are highly activated and start to proliferate - a pre-latency period characterized by cell-doublings every 8-12 h and preceding transformation. We hypothesized that significant metabolic adaption is needed at 96 hpi for infected B cells to enter cell cycle and initiate the hyper-proliferative phase.

At 96 hpi, quinolinate (QUIN) was the most upregulated metabolite in EBV-infected B cells as compared to h.i. EBV exposed B cells, whilst tryptophane (L-TRYP) and NAD⁺ levels were decreased **(****Figure 2B****).** This suggested activation of the kynurenine pathway (KP) upon EBV infection. Activation of the KP, with IDO1 and QPRT being its rate-limiting enzymes, sequentially catabolizes L-TRYP into QUIN, which can be further utilized for NAD⁺ *de novo* biosynthesis **(****Figure 1****).**

In line with the metabolomic data, RNA-seq analyses revealed that, at 96 hpi, EBV infected B cells upregulated gene transcripts of *IDO1* and *QPRT* by up to 4-fold (**Figure 3A**). Boxed area represents a group of upregulated gene transcripts. Notably, IDO1 protein levels were highest at 96 hpi followed by a sharp decline, whereas QPRT protein was maintained throughout transformation of cells (**Figures 3B-3C**). Protein abundance of the non-rate limiting KP enzymes, such as kynureninase (KYNU), 3-hydroxyanthranilate 3,4-dioxygenase (HAAO) and glutamine-dependent NAD⁺ synthetase (NADSYN1), were not significantly altered early upon infection with EBV **(****Figures 3B-3C****).** Those omics-based findings led us to re-assess the levels of the KP metabolites L-TRYP and L-KYNU by targeted liquid chromatography-mass spectrometry (LCMS/MS). Aligning with the insight gained from our omics-data discovery platform, an increased ratio of L-KYN/L-TRYP was detected, establishing accelerated catabolism of L-TRYP towards L-KYN by elevated IDO1 activity **(****Figure 3D****).**

Together these data identified transient IDO1 expression as signature metabolic adaptation associated with early EBV infection of B cells.

### Example 2 - Kynurenine Pathway (KP) in immunosuppression

Immunosuppression is linked with EBV-reactivation due to reduced immune-control of latently infected cells. Therefore, to explore whether there is evidence of KP activity in immunosuppressed individuals, KP metabolites were longitudinally quantified among solid organ transplant (SOT) recipients enrolled in the prospective Swiss transplant cohort study (STCS). Study participants were stratified into three categories, reflecting a spectrum of EBV immune control, ranging from full control to clinically relevant loss thereof. Specifically, we longitudinally tested serum samples from SOT recipients with (i) no EBV DNA detectable in plasma throughout an observation period of 18 months starting with transplantation (n=10), (ii) EBV DNA repetitively detectable in serum in the 18-month post-transplant observation period, no evidence of post-transplant lymphoproliferative disorder (PTLD) (n=10), and (iii) EBV DNA repetitively detectable and development of biopsy proven PTLD within 18 months of transplantation (n=10). In line with our *in vitro* findings, L-KYNU/L-TRYP and QUIN/L-TRYP ratios increased from cohorts (i) to (ii) and again from cohorts (ii) to (iii) **(****Figure 3E****).**

### Example 3 - cell proliferation

To probe whether transient IDO1 expression upon EBV infection of B cells was a metabolic requirement for latent infection, we monitored EBV-driven B cell proliferation in relation to pharmacologic IDO1 blockade in a first step. Importantly, when adding the irreversible IDO1 inhibitor, BMS-986205, at 0 hpi, EBV driven B cell proliferation was inhibited in a dose-dependent manner **(****Figure 4****).** These data show that EBV-induced IDO-1 activity is required for B cell proliferation.

### Example 4 - Transient IDO1 expression and EBV infection of B cells

To probe whether transient IDO1 expression upon EBV infection of B cells was a metabolic requirement for latent infection (and hence B cell transformation), a bespoke assay was developed to monitor EBV-driven B cell transformation in relation to pharmacologic IDO1 blockade. Importantly, when adding the irreversible IDO1 inhibitor, BMS-986205, at 0 hpi, EBV driven B cell transformation was efficiently suppressed - strongly supporting that early IDO1 expression and activity was a metabolic requirement of B cell transformation.

To confirm the requirement of EBV-induced IDO1 activity for EBV-driven B cell transformation, we next assessed whether metabolites downstream of IDO-1 could restore the transformation capacity of EBV. Indeed, addition of L-KYNU partially restored EBV's capacity to latently infect B cells, whereas the direct precursor of NAD⁺, NaMN, fully restored this capacity **(****Figure 5A**). In fact, addition of NaMN even slightly increased transformation efficiency compared to vehicle. These data established the importance of early, transient IDO-1 activity in nascent EBV infected B cells fueling NAD⁺ *de novo* biosynthesis as a metabolic requirement of EBV latent infection/B cell transformation. The observation that L-KYNU only partially restored EBV's transformation efficiency suggests that QPRT represents a significant bottle neck in the flux towards NAD⁺ *de novo* biosynthesis in this biologic system.

To further solidify the findings made using BMS-9866205, we also tested Epacadostat, another IDO1 inhibitor. Likewise, addition of Epacadostat concurrent with EBV infection (i.e. at 0 hpi) efficiently suppressed transformation of EBV-infected B cells **(****Figure 5B**). Also in this setting, simultaneous addition of NaMN fully rescued transformation of B cells in the presence of the inhibitor **(****Figure 5B**).

In summary, these data identify a metabolic vulnerability of EBV in the process of establishing latency in B cells - which is a prerequisite for malignant B cell transformation. Specifically, we show the activation of IDO1 is critical in this process. Pharmacologic blockade of IDO1 very significantly hindered EBV from establishing latency in B cells - and thus driving B cell transformation. Addition of the NAD⁺ precursor L-KYNU partially and dose-dependently rescued the capacity of EBV to transform IDO1-blocked B cells, whereas the direct NAD⁺ precursor, NaMN, was able to fully rescue IDO1-blockade.

These data thus demonstrate that IDO1 plays a key role in EBV transformation of primary B cells. IDO1 inhibitors can therefore be used to prevent naive B cells from becoming infected with EBV, to prevent newly infected B cells from becoming latently infected and to suppresses the transformation of EBV-infected cells and therefore treat or prevent a range of EBV-associated pathologies.

## Claims

1. An Indoleamine 2,3-dioxygenase 1 (IDO1) inhibitor for use in a method of treating an Epstein-Barr virus (EBV) associated disease or condition in a subject.

2. The IDO1 inhibitor for the use of claim 1, wherein the IDO1 inhibitor is a small molecule IDO1 inhibitor, a vaccine or a shRNA.

3. The IDO1 inhibitor for the use of claim 1 or 2, wherein the IDO1 inhibitor is a small molecule IDO1 inhibitor and is selected from the group consisting of hydroxyamidines, 1-(4-arylcyclohex-1-yl)propanamides, Indole and [5,6]-fused heteroaromatics, Phenylimidazoles, 1,2-diamino- and 1-hydroxy-2-amino-substituted aromatics; and pharmaceutically acceptable salts thereof.

4. The IDO1 inhibitor for the use of claim 3, wherein the IDO1 inhibitor is a hydroxyamidine or a pharmaceutically acceptable salt thereof.

5. The IDO1 inhibitor for the use of claim 4, wherein the IDO1 inhibitor is Epacadostat (INCB024360) or a pharmaceutically acceptable salt thereof.

6. The IDO1 inhibitor for the use of claim 3, wherein the IDO1 inhibitor is a 1-(4-arylcyclohex-1-yl)propenamide or a pharmaceutically acceptable salt thereof.

7. The IDO1 inhibitor for the use of claim 6, wherein the IDO1 inhibitor is Linrodostat (BMS 986205) or a pharmaceutically acceptable salt thereof.

8. The IDO1 inhibitor for the use of claim 3, wherein the IDO1 inhibitor is a 1,2-diamino-or 1-hydroxy-2-amino-substituted aromatic or a pharmaceutically acceptable salt thereof.

9. The IDO1 inhibitor for the use of claim 8, wherein the IDO1 inhibitor is KHK2455 or a pharmaceutically acceptable salt thereof.

10. The IDO1 inhibitor for the use of any one of the preceding claims, wherein the EBV associated disease or condition is selected from post-transplant lymphoproliferative disorder (PTLD), Infectious Mononucleosis (IM) or glandular fever, chronic active EBV (CAEBV), haemophagocytic syndrome (HPS), hemophagocytic lymphohistiocytosis, immune haemolytic anemias, an EBV associated cancer, an immunodeficiency, and an EBV associated autoimmune disease.

11. The IDO1 inhibitor for the use of any one of the preceding claims, wherein the EBV associated disease is PTLD or IM, preferably PTLD.

12. The IDO1 inhibitor for the use of any one of claims 1-10, wherein the EBV associated cancer is a lymphoma, preferably derived from B cells.

13. The IDO1 inhibitor for the use of claim 12, wherein the EBV associated cancer is a lymphoma selected from immunoblastic lymphoma, Burkitt's lymphoma, Hodgkin's lymphoma, NK cell lymphoma, T cell lymphoma, diffuse large B cell lymphoma and primary effusion lymphoma; or a carcinoma selected from nasopharyngeal carcinoma and gastric carcinoma.

14. The IDO1 inhibitor for the use of any one of claims 1-10, wherein the immunodeficiency is selected from Ataxia-Telangiectasia, ITK deficiency, X-linked lymphoproliferative disease (XLP), Wiskott-Aldrich syndrome, CD27 deficiency, XMEN disease (MAGT1 deficiency), Coronin 1a deficiency, autoimmune lymphoproliferative syndrome (ALPS), *MST1* mutation (STK4 deficiency), Omenn syndrome, DiGeorge syndrome, Activated PI3K-δ syndrome, WHIM syndrome, CTPS1 deficiency, MCM4 deficiency, ZAP70 deficiency and NF-κB1 haploinsufficiency.

15. The IDO1 inhibitor for the use of any one of claims 1-10, wherein the EBV associated autoimmune disease is selected from multiple sclerosis, systemic lupus erythematosus, rheumatoid arthritis and inflammatory bowel disease.

16. The IDO1 inhibitor for the use of any one of claims 1-9, wherein the method comprises preventing post-transplant lymphoproliferative disorder (PTLD) in a subject.

17. A method of treating an EBV associated disease as defined in any of the preceding claims in a subject in need thereof, comprising administering to the subject a therapeutically effective amount or a prophylactically effective amount of an IDO1 inhibitor as defined in any of the preceding claims or a composition comprising an IDO1 inhibitor as defined in any of the preceding claims.
